(19) Europäisches Patentamt
European Patent Office
Office européen des brevets

(11) **EP 1 482 305 A1**

(12) **EUROPEAN PATENT APPLICATION**
published in accordance with Art. 158(3) EPC

(43) Date of publication:
01.12.2004 Bulletin 2004/49

(51) Int Cl.[7]: **G01N 23/223**

(21) Application number: 02731028.3

(86) International application number:
**PCT/RU2002/000048**

(22) Date of filing: 14.02.2002

(87) International publication number:
**WO 2003/069321 (21.08.2003 Gazette 2003/34)**

(84) Designated Contracting States:
**AT BE CH CY DE DK ES FI FR GB GR IE IT LI LU MC NL PT SE TR**

(71) Applicant: Kumakhov, Muradin Abubekirovich
Moscow, 123298 (RU)

(72) Inventor: Kumakhov, Muradin Abubekirovich
Moscow, 123298 (RU)

(74) Representative: Zellentin, Rüdiger, Dr.
Zweibrückenstrasse 15
80331 München (DE)

(54) **DEVICE FOR IMAGING THE INTERNAL STRUCTURE OF AN OBJECT**

(57) Device comprises X-ray tube 1 with planar through-target anode 2. Between it and the object 8 investigated is situated first collimator 5 with hole-type channels 4. Means 16 comprising detector array 17 registers secondary radiation excited in substance of the object investigated. Between the latter and means 16 is situated second collimator 9 with hole-type or slotted 23 channels. Position of emitting spot of tube 1 on the surface of its anode 2 is scanned by control means 6 of electron beam. Spot 3 is positioned in turn before inlets of different channels of collimator 5. At that, at the output of one of detectors of means 16 appears signal carrying information on substance density of the object 8 in the zone of intersection of the channel of first collimator having at its inlet radiation from tube 1, and continuation of one of channels of second collimator 9. This signal arrives at the input 18 of unit 19.1 of information processing belonging to means 19 of information processing and representation. Information on the current position of electron beam and, consequently, of emitting spot 3 arrives at the input 20 of unit 19.1 belonging to means 6 (in other embodiment such information is contained in modulation of radiation from tube 1, while link shown between means 6 and unit 19.1 is absent). From these data and known mutual arrangement of channels of collimators 5 and 9, substance density distribution in all volume of the object 8 is determined in unit 19.1. Results are displayed as two-dimensional or volume pattern 21 on monitor 19.2 belonging to means 19. Device may comprise also additional collimator 9a and means 16a with detectors 17a, as well as means 28 for detecting radiation of tube 1 transmitted through the object 8 and generation of shadow projection 29 of its internal structure.

fig. 1

## Description

Field of the invention

The inventions proposed relate to the means of diagnostic imaging and are intended for generation in a visually perceptible form of an image of internal structure of an object, in particular, biological one, using X-ray radiation. Preferred fields of application of the inventions proposed include nondestructive testing and medical diagnostics.

Background of the invention

Different devices designated for the purpose specified are known, which realize traditional principles of the projection roentgenoscopy. In such devices, the visible image of the internal structure of an object, for example, tissues of biological object, are acquired as a shadow projection. Density of the image generated in each point is determined by total attenuation of X-ray radiation penetrating object investigated on the way from source to detecting means. As latter is used a fluorescent screen or X-ray film, which is subjected to chemical treatment for image visualization (see · Polytechnical dictionary, Moscow, "Sovetskaya Entsiklopediya" publishing house, 1976 [1], p.425; Physics of images visualization in medicine. S.Webb, Ed., Moscow, "Mir" publishing house, 1991 [2], p.40-41 ).

In the known devices named the image of real three-dimensional structure is obtained in the form of two-dimensional shadow projection mentioned, interpretation of which requires corresponding experience or qualification of specialist performing analysis of the object, in particular, technical or medical diagnostics, and in a number cases is difficult. This is caused by poor contrast, low signal/noise ratio, inevitable superposition of images of the structural elements, and impossibility of quantitative comparison of separate local fragments of the object in density. Sharpness and contrast of the image acquired decrease also in the result of quanta of secondary scattered Compton radiation hitting the detecting means.

The devices are known for X-ray computed tomography allowing to obtain two-dimensional image of a thin layer of three-dimensional object (V.V.Piklov, N. G.Preobrazhensky. Computed tomography and physical experiment. Uspekhi Fizicheskikh Nauk (Progress in Physical Sciences), vol.141, No.3, November 1983, pp. 469-498 [3]; see also [2], pp.138-146). In such devices, repeated irradiation of the test object is performed from different positions and registration of radiation penetrating the object with a detector ruler. Density distribution of the object tissues in the cross section studied is recovered in discrete form by computer-aided solving of equations set with order and number of resolution elements corresponding to product of the number of positions, from which irradiation is performed, on the number of detectors. By performing irradiation in different cross sections, it is possible to obtain three-dimensional image of the object on basis of two-dimensional layer-by-layer images. Computerized tomography means allow in principle to obtain an image of rather high quality. At that, it represents indeed the image of tissues density distribution, rather than pattern resulting from the integral absorption of substance (for example, biological tissues) located in the way of radiation from the source to this or another element of the projection observed. However, this is achieved at the cost of increase in the number of positions, from which the irradiation is performed. At that, increases the dose of radiation absorbed by the substance, which is undesirable (and in medical applications most often unacceptable). Existence of the Compton scattered radiation also constitutes negative factor in this group of known devices. Besides, it is characteristic for medical applications of the devices of both groups considered that tissues and organs situated in the way of radiation (both in front of and behind the region studied), while being of no interest for the study, are also subjected to intensive irradiation. In the devices of the second group radiation level is below that for the devices of the first group as a result of different tissues and organs surrounding those studied being irradiated on choosing different positions.

Increase in resolving ability in the devices of second group, requiring increase in the number of exposures from different positions, is limited, in the first place, by inadmissible growth of the radiation doses. Technical means for recovery of primary information and subsequent image reconstruction are rather complex. This is conditioned both by need in utilization of high-performance computers with specific software and by high requirements to precision of mechanical constructional elements which should guarantee accurate localization of one and the same resolution elements in the region studied on their exposure from different positions. The latter is stipulated by the necessity of actual data appearing in computations on image reconstruction, acquired in different exposure cycles but relating to one and the same resolution elements.

Devices are also known for diagnostic imaging (international application PCT/RU 00/00207 - international publication WO 01/59439 of 16.08.2001 [4]), in which X-ray radiation from a source, in particular, extended source, is concentrated by means of one or several X-ray lenses or collimators in a zone located inside the investigated region of the object. Secondary scattered radiation (Compton, fluorescent) arising in the zone is transported (also using one or several X-ray lenses or collimators) to one or several detectors. Scanning of the investigated region of the object is achieved by shifting of radiation concentration zone. By the aggregate of intensity values of secondary radiation acquired by means of one or several detectors and being determined simultaneously with coordinates locating center of the zone specified, it is possible to assess density of the object substance in said zone. By the aggregate of found density values "bound" to coordinates of radiation concentration zone in its different discrete positions, pattern of the substance density distribution in the region investigated of the object is drawn in means of information processing and mapping.

Principle of operation of this group of the devices is based on the fact of intensity of secondary scattered Compton radiation (expectancy of quanta appearance of this radiation), other things being equal (in particular, at a given intensity of primary X-ray radiation acting on the substance), being proportional to the density of substance. Thus, as distinct from the two previously described groups of devices, in the devices belonging to this group secondary scattered radiation transforms from interfering into informative factor. These devices when applied in medical purposed are characterized also with lower radiation exposure of tissues and organs surrounding those investigated.

However, realization of these advantages is associated with mechanical scanning of X-ray optical system comprising source of radiation, means for its concentration, means for transportation of secondary radiation appearing to the detectors and detectors themselves. Besides, in the course of device operation in each time point just that information is used which is contained in the secondary radiation from the zone of concentration of the source radiation, while useful information containing in secondary radiation excited on propagation of radiation from the source to concentration zone. As a result, no potential capabilities are realized of lowering radiation exposure of the object investigated.

The devices belonging to the latter of groups named above from source [4] (namely, the devices with extended X-ray source and collimators used for transportation both of source radiation to the object investigated and secondary radiation to the detectors) in which information is obtained on density of substance of the object investigated in discrete resolution elements, are the most close to the proposed ones.

Disclosure of the inventions

Technical result, at the attainment of which the inventions proposed are aimed, consists in elimination of mechanical scanning, while retaining excited secondary radiation (scattered Compton coherent and incoherent radiation, fluorescent radiation) as informative factor. The given result is combined with increase in information content and lowering of radiation exposure of the object investigated caused by utilization of information on the intensity of secondary radiation practically simultaneously (due to non-mechanical scanning) in all the volume of source radiation action on the object studied. In addition, technical result is achieved consisting in expedient combining of the above principle of operation based on utilization of secondary radiation, with possibility of generating usual shadow projection. One more kind of technical result, at the attainment of which are aimed the inventions proposed, comprises achievement of such combination of the design and principle of operation of the devices, which facilitates registering of absorbance of primary and excited in the substance of the object studied secondary radiation, thus increasing precision.

The inventions proposed are represented by two embodiments.

To attain the kinds of technical result specified the device proposed for imaging of the internal structure of an object in the first embodiment, similar to the most close known device, comprises extended X-ray source, means for positioning of the object studied, means for detecting of secondary radiation excited in the object studied, and means for information processing and representation. The latter is made with possibility of determination of the substance density in different volume parts of the object investigated and representation of the substance density distribution in the object. Output of means for detecting of secondary radiation excited in the object studied is connected to the input of means for information processing and representation. The proposed and the most close to it known device have also in common the fact of its containing two multichannel collimators. One of them is situated between the extended X-ray source and the means for positioning of the object studied, and the other - between said means and means for detecting of secondary radiation excited in the object studied.

As distinct from the most close known device, in the device proposed extended X-ray source consists of X-ray tube with planar through-target anode, made with possibility of formation of emitting spot in turn before inlets of different channels of the first collimator by corresponding scanning of electron beam of the X-ray tube with control means for this beam.

The latter has an output of information signal on the current position of electron beam, connected to the second input of means for information processing and representation.

The first collimator is an aggregate of parallel, convergent or divergent hole-type channels, while second collimator is an aggregate of parallel, convergent or divergent, hole-type or slotted channels. Both collimators are made and oriented in such a way that channels continuations of the first collimator intersect with channels continuations of the second collimator in zone intended for positioning of the object studied. At that, they form intersection regions comprising nodal points of imaginary lattice having cells in the form of convex hexahedrons.

Second collimator is preferably made with parallel, hole-type or slotted channels. In this case, cells of the imaginary spatial lattice specified have a form of parallelepipeds or truncated rectangular pyramids with parallel bases.

Means for detecting of secondary radiation excited in the object investigated is an aggregate of detectors, each made and installed with possibility of reception of output radiation of just one channel of the second collimator from among those having it at the output simultaneously.

Means for information processing and representation is made with possibility of obtaining information on the substance density in each element of volume of the object studied, consisting of intersection region of channels continuations of first and second collimators comprising only one of nodal points of imaginary spatial lattice specified. For this, it is made with possibility of using intensity of the secondary radiation excited in said volume element of the object investigated during its irradiation, registered with corresponding detector of the means for detecting secondary radiation excited in the object studied, as a value proportional to the density sought.

Besides, means for information processing and representation may be made with possibility of allowance for attenuation of radiation from the extended X-ray source on the way to volume element specified of the object studied. For this, said means is made with possibility of computation of said attenuation taking into account previously determined values of the substance density in other elements of volume of the object investigated located closer to the outlet of corresponding channel of the first collimator on intersects of continuation of said channel with continuations of other channels of second collimator.

Means for information processing and representation may be made also with possibility of accounting for attenuation of said excited secondary radiation in the course of its passage through the object investigated in the direction of corresponding detector, when obtaining information on the substance density in each of said volume elements. For this, said means is made with possibility of computation of said attenuation with allowance for previously determined values of substance density in other volume elements of the object studied situated closer to inlet of the same channel of second collimator on intersects of continuation of this channel with continuations of other channels of the first collimator.

For more precise imaging of the internal structure of the object studied the device proposed may comprise at least one additional collimator, made and arranged subject to the same conditions, as the second collimator. In this case, the device proposed comprises also corresponding additional means for detecting of secondary radiation excited in the object investigated. It is situated at the outlets of the channels of the additional collimator and is attached to the means for information processing and representation. The latter is made with additional possibility for processing of output signals from the additional means for detecting secondary radiation excited in the object investigated, as well as output signals from the means for detecting of secondary radiation excited in the object investigated, situated at the outlets of channels of the second collimator.

At that, additional collimator may be both identical to the second collimator and different from it (for example, second collimator may have parallel hole-type channels, and the additional one - parallel slotted channels).

Means for information processing and representation in the presence of additional collimator may be made with possibility of formation of the substance density values in different volume elements of the object investigated, using as previously determined values of substance density in other volume elements of the object investigated the values found as a result of co-processing of output signals from all the means for detecting secondary radiation excited in the object investigated.

To realize possibilities of generating usual shadow projection, the device proposed may comprise means for detecting radiation from extended X-ray source passing through the object investigated, located behind the means for positioning of the object investigated.

The means for detecting radiation from extended X-ray source passing through the object investigated may be made either immediately generating visible image, or with electric output attached to the means for information processing and representation. In this case, the latter is made with possibility of formation and visualization of shadow projection image of the object investigated.

The device proposed according to second embodiment, similar to the device most close to it as described in the reference source [4], comprise extended X-ray source, means for positioning of the object investigated, means for detecting secondary radiation excited in the object investigated, two multichannel collimators, one being situated between extended X-ray source and means for positioning the object investigated, and the other - between this means and the means for detecting secondary radiation excited in the object investigated, as well as means for information processing and representation. The latter is made with possibility of determination of the substance density in different parts of volume of the object investigated and visualization of the substance density distribution in the object. Inlet of this means is attached to the output of means for detecting secondary radiation excited in the object investigated.

As distinct from the most close device, the device proposed according to second embodiment disclosed consists of X-ray tube with a planar through-target anode. This tube is made with possibility of emitting spot formation in turn before inlets of different channels of the first collimator and imparting at that to X-ray radiation of an attribute carrying information on the current position of the emitting spot by corresponding scanning of electron beam of the X-ray tube and modulation of the radiation with means of the beam control incorporated in the device.

The first collimator is an aggregate of parallel, convergent or divergent hole-type channels. The second collimator is an aggregate of parallel, convergent or divergent hole-type or slotted channels. Both collimators are made and oriented in such a way that continuations of channels of the first collimator intersect with continuations of channels of the second collimator in a zone intended for positioning of the object investigated. At that, they form intersection regions comprising nodal points of imaginary spatial lattice with cells in the form of convex hexahedrons.

The means for detecting secondary radiation excited in the object investigated is an aggregate of detectors, made and installed each with possibility of receiving output radiation from only one channel of the second collimator from the number of those having it at the outputs simultaneously.

The means for information processing and representation is made with possibility of demodulation of output signals from the detectors of the means for detecting secondary radiation excited in the object investigated, and obtaining information on the current position of the emitting spot. It is made also with possibility of obtaining information on substance density in each element of volume of the object investigated formed by the intersection region of continuations of channels of first and second collimators comprising just one of nodal points of said imaginary spatial lattice, from the intensity of secondary radiation excited in said volume element of the object investigated during its irradiation, registered with corresponding detector of the means for detecting secondary radiation excited in the object investigated.

Control means of electron beam may be made, in particular, with possibility of pulsed modulation of X-ray tube radiation, with changing at that of repetition rate or duration of impulses in accordance with current position of the electron beam.

In other respects, the device proposed in the second embodiment doesn't differs from the device by the first embodiment in all the above described particular cases of embodiment.

Brief description of drawings

The inventions proposed are illustrated with drawings, in which:

- Fig.1 shows schematic representation of relative positioning and connection of principal units of the device proposed in case of the first and second, as well as additional collimators, made with parallel hole-type channels;

- Fig.2 shows fragment of imaginary spatial lattice for the case illustrated in Fig.1;

- Fig.3 shows schematic representation of relative positioning and connection of principal units of the device proposed in case of the first collimator made with parallel hole-type, and the second and additional collimators - with parallel slotted channels;

- Fig.4 shows fragment of imaginary spatial lattice for the case illustrated in Fig.3;

- Fig.5 shows relative positioning of collimators' channels and means for detecting secondary radiation, as well as volume elements of the object investigated, substance density in which is taken into consideration in accounting for the attenuation of radiation from extended X-ray source and secondary radiation, in the device of Fig. 1;

- Fig.6 shows relative positioning of collimators and means for detecting secondary radiation, as well as volume elements of the object investigated, substance density in which is taken into consideration in accounting for the attenuation of radiation from extended X-ray source and secondary radiation, in the device of Fig. 3;

- Fig.7 shows schematic representation of relative positioning and connection of principal units of the device proposed in case of the first collimator being made with convergent, and the second and additional collimators - with parallel hole-type channels;

- Fig.8 shows schematic representation of relative positioning and connection of principal units of the device proposed in case of the first collimator being made with convergent hole-type, and the second and additional collimators - with parallel slotted channels;

- Fig.9 shows schematic representation of relative positioning and connection of principal units of the device proposed in case of the first collimator being made with divergent, and the second and additional collimators - with parallel hole-type channels;

- Fig.10 shows schematic representation of relative positioning and connection of principal units of the device proposed in case of the first collimator being made with divergent hole-type, and the second and additional collimators - with parallel slotted channels;

- Fig.11 shows a fragment of imaginary spatial lattice for the case illustrated in Fig.7;

- Fig.12 shows a fragment of imaginary spatial lattice for the case illustrated in Fig.8;

- Fig.13 shows a fragment of imaginary spatial lattice for the case illustrated in Fig.9;

- Fig.14 shows a fragment of imaginary spatial lattice for the case illustrated in Fig.10;

- Fig.15 shows schematic representation of intersections of continuations of divergent channels of the first collimator with those of divergent slotted channels of the second collimator;

- Fig.16 and Fig. 17 show schematic representation of intersections of continuations of the channels of first and second collimators located on one and the same side of a large-sized object;

- Fig.18 shows schematic representation of relative positioning and connection of principal units of the device proposed in case of the first collimator being made with parallel hole-type, and the second and additional collimators - with parallel slotted channels, in the embodiment of the device requiring no connection of control means of the electron beam with means for information processing and representation.

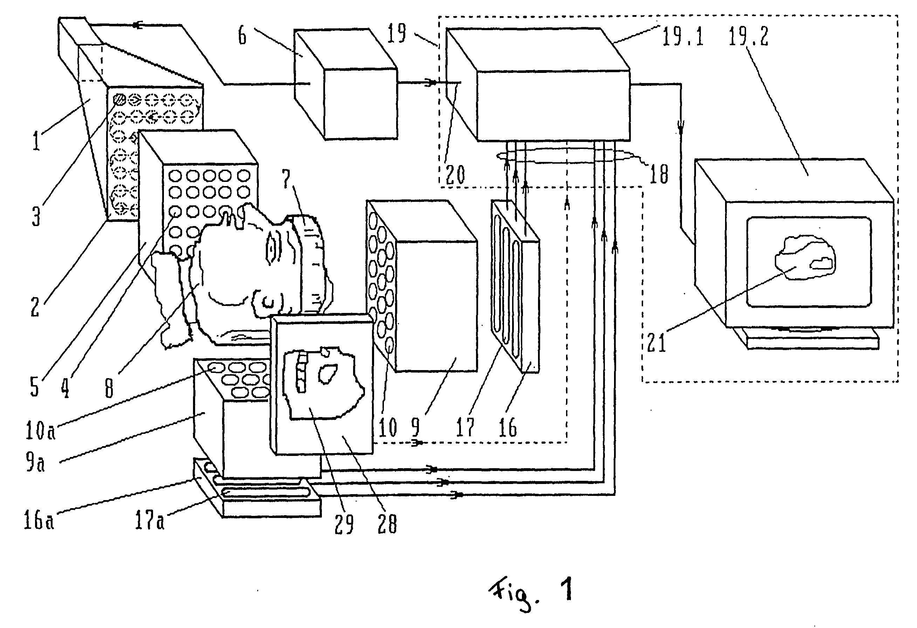

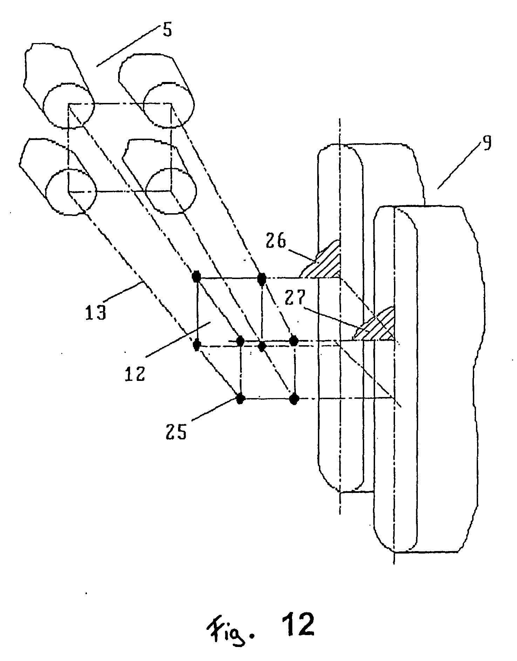

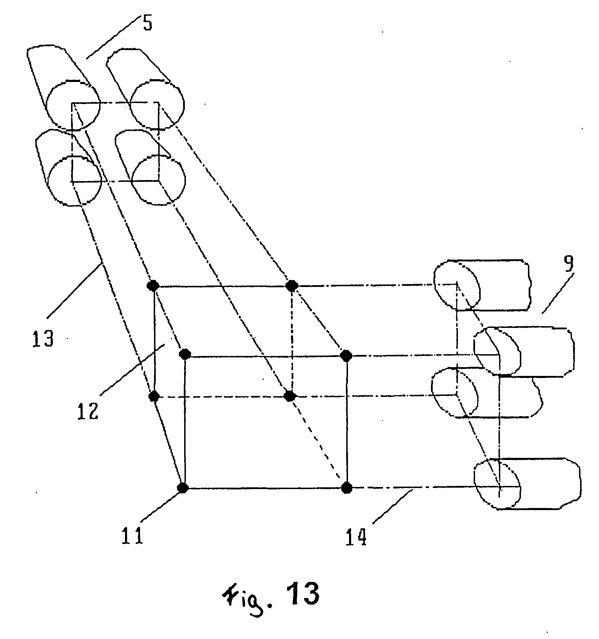

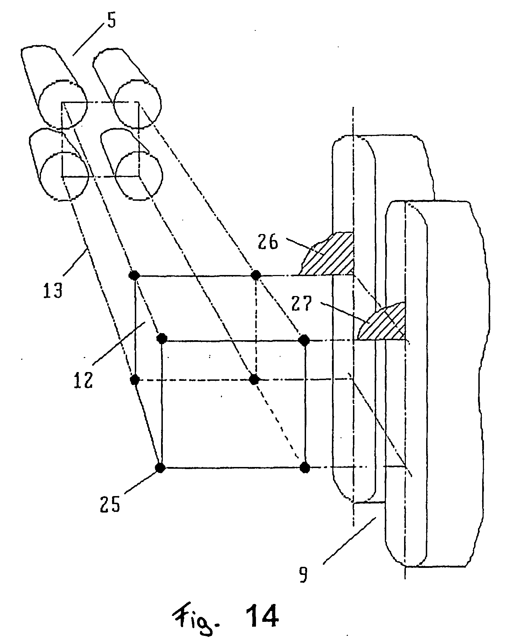

Figs.1-17 relate to the first embodiment of the invention. These same drawings, except for connection between control means of the electron beam and means for information processing and representation in Figs.1, 3 and 7-10, relate also to the second embodiment of the invention.

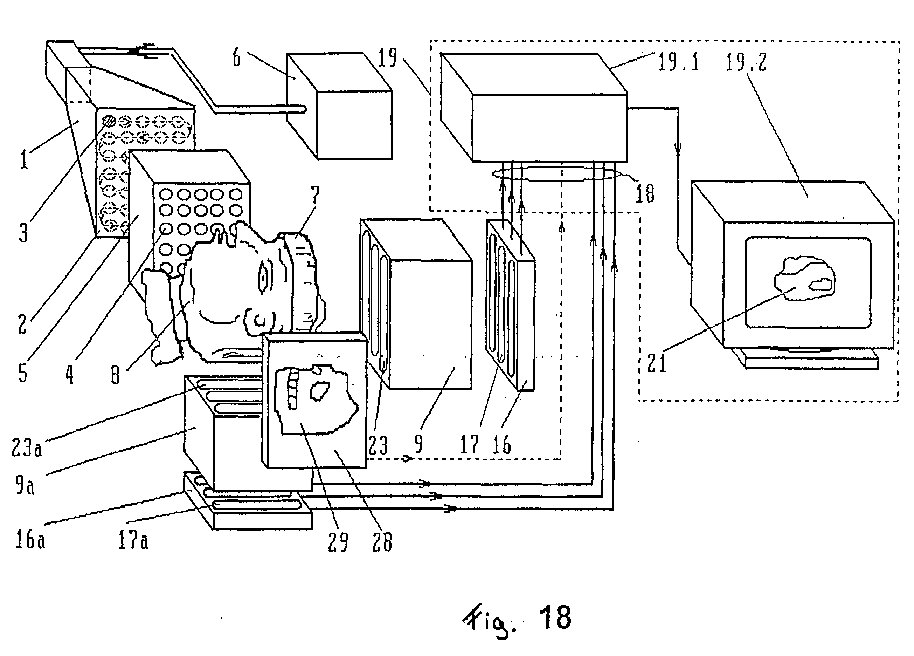

Fig. 18 relates to the second embodiment of the invention.

The drawings specified use following designations:

| | |
|---|---|
| 1 - | X-ray tube |
| 2 - | anode of X-ray tube |
| 3 - | emitting spot on the external surface of through-target anode of X-ray tube |
| 4 - | hole-type channel of the first collimator |
| 4.1- 4.4 - | specific channels of the first collimator in Figs.2, 4 |
| 4.5 - 4.9 - | specific channels of the first collimator in Fig.5 |
| 4.10, 4.11 - | specific channels of the first collimator in Fig.6 |
| 5 - | first collimator |
| 6 - | control means of electron beam of the X-ray tube |
| 7 - | means for positioning of the object investigated |
| 8 - | object investigated |
| 9 - | second collimator |
| 9a - | additional collimator |
| 10 - | hole-type channel of the second collimator |
| 10.1 - 10.4 - | specific channels of the second collima- |

| | |
|---|---|
| | tor in Fig.2 |
| 10.5 - 10.6 - | specific channels of the second collimator in Fig.5 |
| 10a - | hole-type channel of the additional collimator |
| 11 - | nodal point of imaginary spatial lattice in the case of hole-type channels of the second collimator |
| 11.1 - 11.8 - | specific nodal points of imaginary spatial lattice in Fig.2 |
| 12 - | cell of imaginary spatial lattice |
| 13 - | continuation of centerline of the channel of first collimator |
| 14 - | continuation of centerline of the channel of second collimator |
| 15 - | volume element of the object investigated in case of hole-type channels of both collimators |
| 15.1 - | volume element of the object investigated in Fig.5, situated closer than others to the outlet of first collimator and to the inlet of second collimator |
| 15.2 - | volume element of the object investigated in Fig.5, for which attenuation of primary radiation takes place in other elements situated between said element and the outlet of the channel of first collimator, and attenuation of secondary radiation in elements situated on the way to the inlet of second collimator |
| 16 - | means for detecting secondary radiation |
| 16a - | additional means for detecting secondary radiation |
| 17 - | separate linear detector of the means for detecting secondary radiation |
| 17.1, 17.2 - | specific linear detectors of Fig.5 |
| 17a - | separate linear detector of the additional means for detecting secondary radiation |
| 18 - | data entry of the means for information processing and representation |
| 19 - | means for information processing and representation |
| 19.1 - | information processing unit |
| 19.2 - | monitor |
| 20 - | second entry of the means for information processing and representation |
| 21 - | image of the internal structure of the object investigated on monitor screen |
| 22.1, 22.2 - | vertical planes passing through centerlines of columns of hole-type channels of second collimator in Fig.2 |
| 23 - | slotted channel of second collimator |
| 23.1, 23.2 - | specific slotted channels of second collimator in Fig.4 |
| 23.3, 23.4 - | specific slotted channels of second collimator in Fig.6 |

23a - slotted channel of additional collimator

24 - volume element of the object investigated in case of hole-type channel of the first collimator and slotted channel of the second collimator

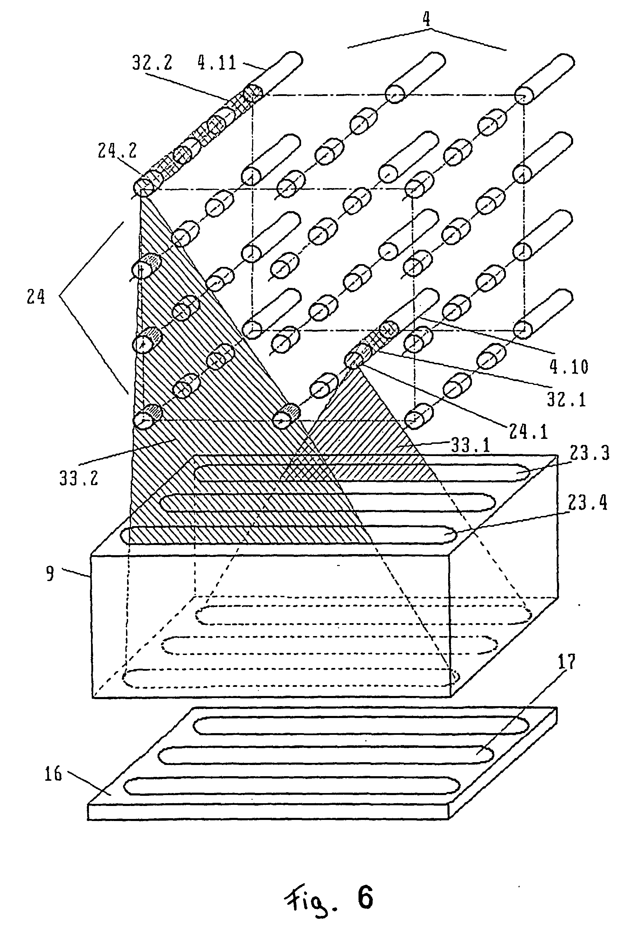

24.1 - volume element of the object investigated in Fig.6, situated closer than others to the outlet of first collimator and to the inlet of second collimator

24.2 - volume element of the object investigated in Fig.6, for which attenuation of primary radiation takes place in other elements situated between said element and the outlet of the channel of first collimator, and attenuation of secondary radiation in elements situated on the way from this element to the inlet of second collimator

25 - nodal points of imaginary spatial lattice in case of slotted channels of the second collimator

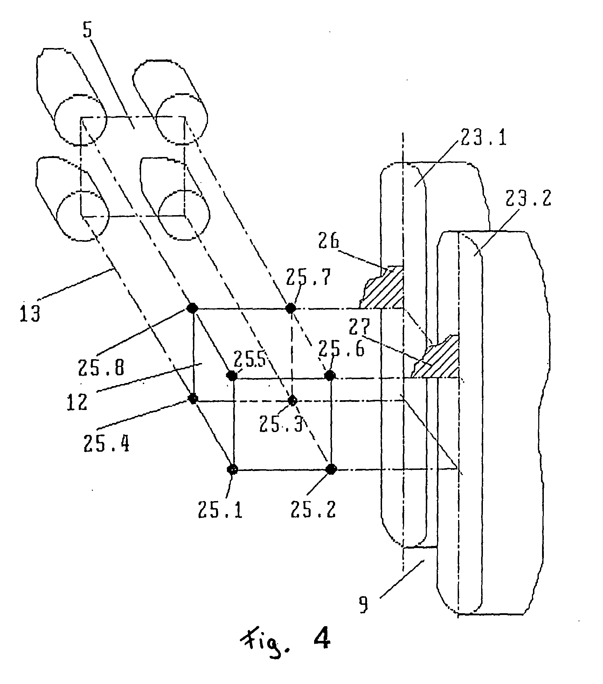

25.1 - 25.8 - specific nodal points of the imaginary spatial lattice in Fig.4

26, 27 - vertical planes passing in the middle between parallel walls of slotted channels of the second collimator

28 - means for detecting secondary radiation passing through the object investigated

29 - shadow projection of the object investigated

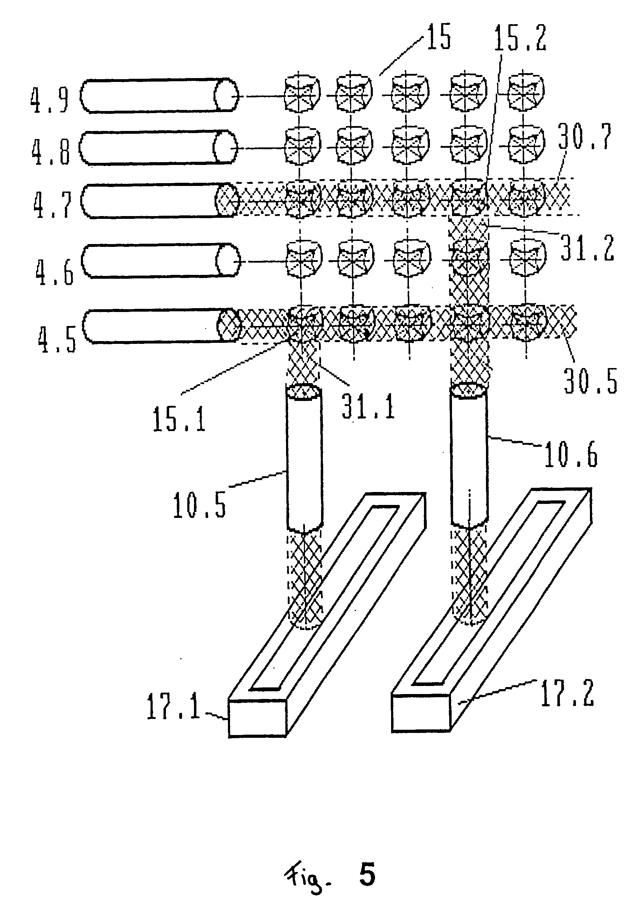

30.5, 30.7 - streams of primary X-ray radiation coming out of channels 4.5 and 4.7 of the first collimator in Fig.5, correspondingly

31.1, 31.2 - parts of the streams of secondary radiation captured by hole-type channels of second collimator in Fig.5

32.1, 32.2 - streams of primary X-ray radiation coming out of channels 4.9 and 4.10 of the first collimator in Fig.6, correspondingly

33.1, 33.2 - parts of the streams of secondary radiation from volume elements 24.1 and 24.2, correspondingly, captured by slotted channels 23.3 and 23.4 of second collimator in Fig.6

Embodiments of the inventions

The device proposed in accordance with first variant of the invention in one of its most preferred embodiments is shown in Fig.1.

It comprises extended X-ray source in the form of X-ray tube 1 with planar through-target anode 2. The X-ray tube 1 is made with possibility of emitting spot formation on the external surface of through-target anode 2 in turn in front of inlets of different parallel hole-type channels 4 of the first collimator 5. For this, scanning is performed of electron beam of the X-ray tube. The scanning may be performed, for example, in a line-by-line manner, in particular, by imparting to the beam a series of discrete positions, corresponding to the discrete positions of the emitting spot 3, as shown in Fig.1, with the help of control means 6 for the beam positioning. Output of the control signal from this means (the left one in Fig. 1) is connected with control input of the X-ray tube 1.

The X-ray tube 1 and control means 5 for electron beam positioning may be made, for example, as described in US patents No.4,259,582 [5] (publ. 31.03.81) and No.5,490,197 [6] (publ. 06.02.96).

The device comprises also means 7 (shown tentatively in Fig.1) for positioning of the object investigated 8 (in Fig.1 and most of other drawings biological object, namely, head of patient, is shown as an object investigated). Means 7 for positioning is mounted in such a manner that the object investigated may be placed in the way of X-ray radiation of tube 1 coming out of channels 4 of the first collimator 5.

In vacant place, aside from the zone intended for positioning of the object investigated but as close to it as possible, second collimator 9 is mounted. In the case illustrated in Fig.1 second collimator, similar to the first one, has parallel hole-type channels 10. Under the hole-type channels in the given case are meant holes of arbitrary form with big aspect ratio, i.e. with length exceeding multiply its transverse dimensions, and rectilinear longitudinal axis and generating lines of the walls (for example, cylindrical).

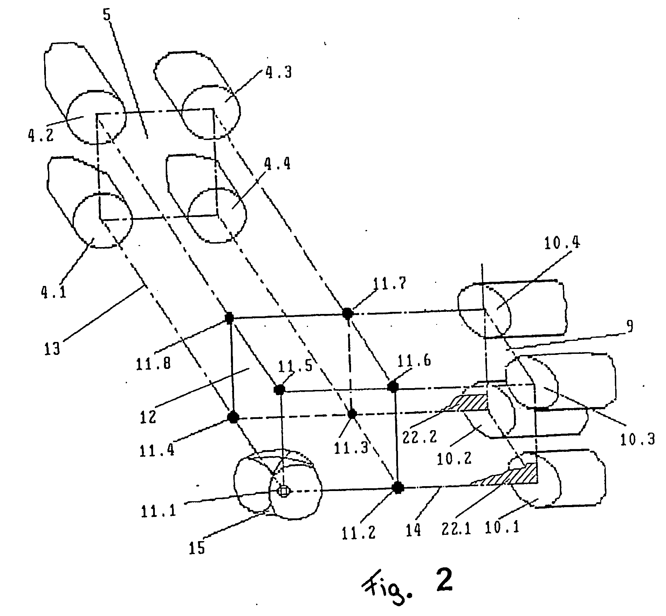

In the case described, channels 4, 10 of both collimators 5, 9 intersect on their continuation towards zone of positioning of the object investigated. At that, they form intersection regions comprising nodal points of imaginary spatial lattice with cells in the form of convex hexahedrons 12. In the case specified, these hexahedrons are rectangular parallelepipeds. Fragment of this lattice is shown in Fig.2. Its nodal points 11 (11.1-11.8) in the given case are located in vertices of the rectangular parallelepiped - intersection points of continuations of centerlines of channels 4 and 10 of the first 5 and second 9 collimators, correspondingly. Fig.2 shows also one of volume elements 15 of the object investigated, corresponding to intersection of continuations of the channels 4 and 10 and comprising nodal point 11.1 of imaginary spatial lattice.

Outlets of the channels 10 of second collimator 9 are turned towards means 16 for detecting secondary radiation excited in substance of the object investigated on the passage through it of primary radiation of X-ray tube 1 directed with first collimator 5.

Means 16 for detecting secondary radiation excited in substance of the object investigated 8 is an aggregate of detectors sensitive to secondary radiation of substance of the object investigated. Each of detectors is made and mounted with possibility of receiving output radiation from only one channel of the second collimator from the number of those having it at the outputs simultaneously.

It is possible, in particular, to make means 16 in the form of matrix with number of detectors equal to the number of channels of second collimator, and positioning of each of the detectors opposite outlet of one of the channels of said collimator. In this case, each detector reacts on output radiation of only one channel of the second collimator.

However, it is more rational to make means 16 in the form of linear detector matrix with aperture of each one of them encompassing outputs of a whole group of channels of the second collimator. In Figs.1 and 2, such groups are vertical columns of channels 10 (10.1-10.4 - Fig.2) of the second collimator 9. Since radiation beam coming out of any channel 4 of the first collimator 5 penetrates the object 8 investigated horizontally and in each time moment is present in one only of channels 4.1-4.4 (Fig. 2), this beam intersects continuations of channels 10.1-10.4 of the second collimator 9 belonging to different vertical columns. Therefore, secondary radiation excited in substance of the object 8 investigated is propagating in each time moment in one only of channels 10.1 - 10.4 of each vertical columns of the second collimator (in Fig.2 - from left to right). Thus, for example, radiation of the X-ray tube from channel 4.1 of the first collimator 5 travels in the direction of longitudinal axis 13 of said channel through nodal points 11.4 and 11.1. Secondary radiation excited in the volume elements comprising nodal points 11.4 and 11.1 (one of these elements - 15 - is shown in Fig.2) may only pass to the outlets of channels of the second collimator 9 located lower in Fig.2 (10.1 and 10.2) and may not pass to the outlets of upper channels 10.3 and 10.4. This renders it possible to make means 16 for detecting secondary radiation in the form of said matrix of linear extended detectors 17, one for each column of channels of the second collimator 9.

Outputs of detectors 17 of the means 16 for detecting secondary radiation are connected to data entry 18 of means 19 for information processing and representation. This means is shown in Fig.1 as unit 19.1 for information processing (with belonging to it input 18) and connected to its output monitor 19.2 performing function of information presentation. Unit 19 of the means for information processing and representation through its second input 20 (belonging to unit 19.1 of information processing) is connected also with data output of control means 6 of electron beam of the X-ray tube 1.

Thus, in the course of operation of the device having design described, signals are coming to unit 19.1 of information processing, which carry information on the current position of electron beam of the X-ray tube 1 (and, consequently, on the position of emitting spot 3 on the surface of its through-target anode 2) and on intensity of secondary radiation excited in the object investigated.

Signals carrying information on intensity of secondary radiation are coming from outputs of different detectors 17. This allows to correlate in the unit 19.1 information on the current position of emitting spot 3 with intensity of secondary radiation in a group of channels (in Fig.1, such group is a column of channels) of the second collimator 9, with outlets being encompassed by aperture of each of extended linear detectors 17. However, as stated above, in each time moment secondary radiation may be present on the outlet of one only of the channels of each column of the second collimator 2. Said channel is a channel in which secondary radiation may propagate at the given moment. Continuation of this channel has intersection inside of the object 8 investigated with continuation of that channel of the first collimator, at the inlet of which arrives radiation from the tube 1. With this information, in combination with information on the current position of the emitting spot 3 (i. e., opposite which of the channels of the first collimator 5 it is situated), in the unit 19.1 exact correspondence may be established between values measured of the intensity of secondary radiation and coordinates of volume elements of the object investigated being sources of secondary radiation.

It is known (J. Jackson. Classical electrodynamics. Moscow, "Mir" publishing house, 1965 (Russian translation) [7]) that the intensity of secondary radiation, other things being equal, is proportional to density of substance in which it arises. Therefore, it follows from the above that for each position the emitting spot occupies in front of the inlet of one of the channels of the first collimator 5, in the unit 19.1 for information processing a set of numbers proportional to density values may be obtained, "tied-in" to the volume elements of the object investigated having known coordinates. As latters, for example, coordinates may be used of nodal points 11 (11.1-11.8) of spatial lattice, fragment of which is shown in Fig.2. If necessary, the device may be calibrated in such a way as to enable estimate of absolute density values in each volume element of the object investigated. However, to obtain pattern of density distribution, it is enough to have relative estimates, i.e. it is enough to obtain and display intensity values for all volume elements of the object investigated on the same scale.

According to the above, function of unit 19.1 of information processing realizing the simple logic described, comes to acquisition of information on substance density in each volume element of the object investigated. Volume element is an intersection region of continuations of the channels of first and second collimators, comprising one nodal point only of said imaginary spatial lattice. For this, it is enough to use in the unit 19.1 of information processing the values of secondary radiation intensity excited in volume element specified of the object investigated during its irradiation as a measure of density. Intensity value is determined by output signal of corresponding detector 17 of the means 16 for secondary radiation detecting.

After scanning of all part of interest of the object investigated, stored values of the secondary radiation intensity adopted as a measure of substance density in volume elements of the object investigated may be used for visualization of this part in any form required. Thus, by selecting intensity values (and corresponding to them coordinate values of volume elements, for example, nodal points 11 of imaginary spatial lattice) for volume elements belonging to any horizontal or vertical layer, it is easy to generate two-dimensional image of this layer. Horizontal layers correspond to horizontal planes passing through channels lines of the first and second collimators in Fig.1, while vertical layers may be of two kinds and correspond to vertical planes passing through columns of channels either of the first or of the second collimator. Generation of these images, as can be seen, requires no calculations and comes down to selection from memory of coordinate sets of lattice nodal points (which are constant for specific device) in combination with corresponding registered values of the secondary radiation intensity. Displaying of such images on monitor 19.2 poses no technical problems and may be realized, for example, similar to method for two-dimensional X-ray images disclosed in patent [6].

Two-dimensional images may be obtained also in different "skew" sections requiring insignificant complication in the selection logics.

Set of data on density obtained in unit 19.1 for information processing in all volume corresponding to intersection of summary apertures of the first and second collimators is sufficient for displaying on monitor 19.2 of three-dimensional image 21 using known methods of computer graphics (see, for example, E.Lapshin. Graphics for IBM PC. Moscow, "Solon" publishing house, 1995 [8] (in Russian)).

Accuracy of density values "tie-in" to coordinates falls in order of dimensions of regions 15 (Fig.2) of intersections of channels continuations of the first and second collimators comprising nodal points 11 shown in Fig.2. With number of channels being sufficiently small (for example, $100 \times 100$ in each collimator) and transverse dimensions of the collimators of the order of $10 \times 10$ cm$^2$, may be obtained resolving power of the order of 1 mm. Means 16 for detecting secondary radiation should only comprise at that 100 linear (extended in one direction) detectors or matrix with 100 rows of ordinary detectors. In the last case, output signals from all detectors in a row are summed and each row functions as one linear detector. Since at present matrices are made with number of detectors substantially greater than that required to obtain such number of rows, device may be realized with substantially higher resolution than that mentioned above. As collimators with corresponding large numbers of hole-type channels, in particular, X-ray grids with cellular structure and parallel channels according to Russian Federation patent No.2,171,979 (publ. 10.08.2001) [9], produced by known technology of monolithic X-ray lens manufacturing (see, for example: V.M.Andreevsky, M.V.Gubarev, P.I.Zhidkin, M.A. Kumakhov, A.V.Noshkin, I.Yu.Ponomarev, Kh.Z.Ustok. X-ray waveguide system with a variable cross-section of the sections. The IV-th All Union Conference on Interaction of radiation with Solids (May 15-19, 1990, Elbrus settlement, Kabardino-Balkarian ASSR, USSR). Book of abstracts. Moscow, 1990, pp. 177-178 [10].

Number of channels of the second collimator may be diminished to the number of columns, with changing at that of their shape to slotted instead of hole-type. Slotted channels are understood as those in form of openings with length exceeding multiply one (smaller) of the transverse dimensions of the channel. Walls of the slotted channel corresponding to its greater transverse dimension are parallel. It is expedient to choose the smaller transverse dimension equal to transverse dimensions of the channels of the first collimator. The other (greater) transverse dimension of the slotted channel should be such as to ensure intersection of continuation of this channel with continuations of the channels of first collimator belonging to one column. At that, dimensions of volume element of the object investigated formed at such intersection are approximately equal in three dimensions. As collimator with slotted channels may be used, in particular, slotted anti-scattering X-ray grid (Chan H.-P., Frank P.H., Doi K., Iida N., Higashida Y. Ultra-High-Strip-Density Radiographic Grids: A New Antiscatter Technique for Mammography. «Radiology», volume 154, Number 3, March 1985, pp. 807—815 [11]).

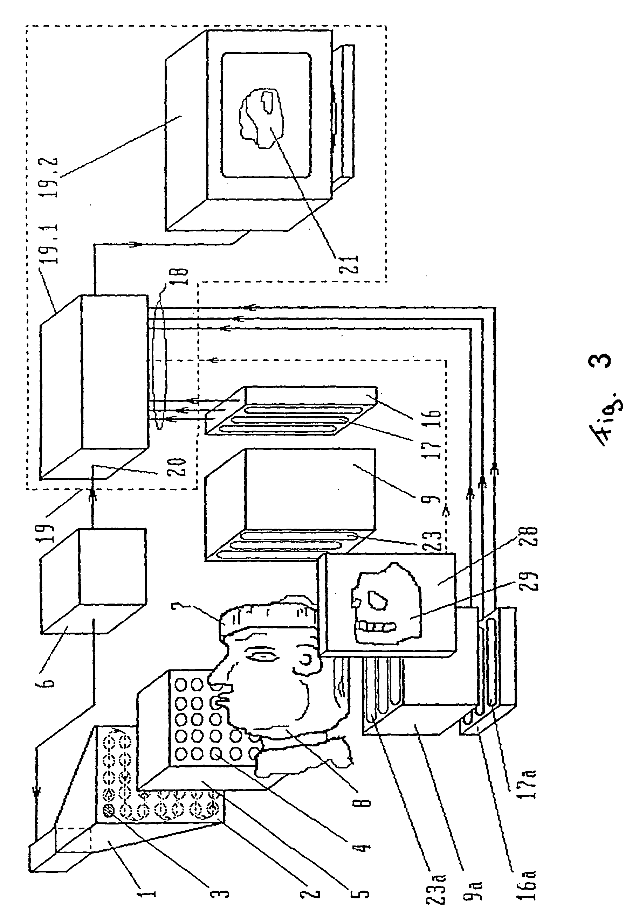

Design of the device proposed, in which second collimator 9 has parallel slotted channels 23, is shown in Fig.3. It is seen from the description of the device operation for the embodiment of Fig.1 above, that determination of specific channel of the given column of channels of the second collimator corresponding to intensity value registered by detector 17 is performed indirectly. For this, it is sufficient to know through which of the channels of the first collimator is irradiated the object investigated at the given moment. Thus, it has been explained above, that in case of the object irradiation through channel 4.1 (Fig.2) secondary radiation may only be present at the outlet of channel 10.2 of one column of channels of the second collimator 9 and at the outlet of channel 10.1 of another column. Position of nodal points 11.1-11.8 is determined in fact by intersection of centerlines 13 of channels of the first collimator 5 with vertical planes 22.1, 22.2 passing through longitudinal axes of channels 10.2, 10.4, and 10.1, 10.3, correspondingly, which form said columns. At that, existence of borders between channels within each of the columns plays no role from the point of view specified, thus making it possible in principle to replace each column of the hole-type channels with one slotted.

Principle of operation of the device with slotted channels of the second collimator doesn't differs from that described above. Changed is only shape of volume element 24 comprising nodal point of imaginary spatial lattice (Fig.4). If in the previous case with circular cross section of the channels of both collimators it was formed by intersection of two cylinders, in the case under consideration it is a part of cylinder (in the case shown in Fig.4 - continuation of channel 4.1 of the first collimator 5) confined between two parallel planes formed by continuations of walls of slotted channel of the second collimator (for volume element shown in Fig.4 - walls of channel 23.2). Nodal points 25 (25.1 - 25.8, Fig. 4) of spatial lattice contained inside volume elements of the object investigated, which are sources of excited secondary radiation, are situated in the intersection points of continuations of centerlines of channels of the first collimator 5 (for example, lines 13 for channel 4.1) with planes 26, 27. The latters pass in the middle between parallel walls of slotted channels 23.1, 23.2 of the second collimator 9.

In both cases shown in Figs 1 and 3, additional collimators 9a may be mounted with hole-type (10a, Fig. 1) or slotted (23a, Fig. 3) channels and additional means 16a of secondary radiation detecting, made and arranged in compliance with the same conditions as second collimator 9 and detecting means 16. In particular, they may be arranged relative to the object investigated in symmetric position with the second collimator 9 and means 16 or in orthogonal position, as shown in Figs.1 and 3, depending on the nature and position of the object investigated. Outputs of detectors 17a of additional means for detecting secondary radiation are also attached to input 18 of unit 19.1 for information processing belonging to means 19 for information processing and representation. In the unit 19.1 for information processing, output signals of detectors 17a are used in the same manner as signals of detectors 17, and values obtained of substance density in volume elements of the object investigated are used together with values obtained from signals of detectors 17 to increase accuracy (for example, by calculating mean values). Additional collimator should not necessarily be identical with the second one. For example, second collimator may have hole-type channels (as collimator 9 in Fig.1), while the additional - slotted channels 23a (as collimator 9a in Fig. 3).

In all the variants of embodiment described, it is possible also to use means 28 for detection of radiation from extended X-ray source penetrating through the object investigated. It is placed in the way of this radiation behind the means for positioning of the object investigated.

Means 28 for detecting radiation of extended X-ray source penetrating through the object investigated may be made either directly generating visible image of shadow projection 28 or with electric output attached to the input of means for information processing and representation. In the first case, means 28 should have a screen with a sufficient (not less than scanning period of electric beam) afterglow. Connection of means 28 with input 18 of unit 19.1 for information processing belonging to means 19 for information processing and representation, existing in the second case, is shown with dotted line. In this case, unit 19.1 for information processing is made with additional possibility of signals shaping for displaying on the monitor 19.2 of shadow projection of the object investigated. Realization of this possibility is totally similar to that described in patent [6].

Two cases of the device embodiment discussed are not equivalent in accuracy. First of all, this is due to the fact of linear detectors 17 in case of Fig.3 being affected with secondary radiation collected by extended slot, rather than separate hole-type channel. For comparison of two device embodiments, while taking this into consideration, Figs.5 and 6 are provided.

Fig.5 shows several hole-type channels (4.5 - 4.9) of the first collimator, two hole-type channels (10.5, 10.6) of the second collimator, two linear detectors (17.1, 17.2) of secondary radiation transmitted through channels 10.5, 10.6, and series of volume elements 15 of the object investigated. Cross-hatched horizontal bars 30.5 and 30.7 designate flux of primary X-ray radiation of tube 1 transmitted through channels 4.5 and 4.7 of first collimator. cross-hatched vertical bar 31.1 corresponds to that part of secondary radiation of volume element 15.1, which may be captured by channel 10.5 of the second collimator, and bar 31.2 - to that part of secondary radiation of volume element 15.2, which may be captured by channel 10.6 of the second collimator.

Fig.6 shows a group of hole-type channels 4 of the first collimator. For two of them (4.10, 4.11) cross-hatching 32.1 and 32.2, correspondingly, shows flux of primary X-ray radiation coming out of these channels. Shown also are second collimator 9 having slotted channels 23 (23.3, 23.4) and means 16 for detecting secondary radiation with linear detectors 17. Small cylinders 24 represent volume elements of the object investigated formed by intersection of continuations of channels 4 of the first collimator and walls of slotted channels 23 of the second collimator. For two volume elements (24.1 and 24.2), hatched sectors 33.1 and 33.2 show part of their secondary radiation able to go through slotted channels 23.3, 23.4 and reach detectors 17.

It follows from comparison of Fig. 5 and Fig. 6 that expected intensity of radiation acting on detectors 17 in the device embodiment according to Fig.3 should be greater than in embodiment of Fig.1. Obviously, due to this the intensity of secondary radiation of volume element may be determined from the output detector signal statistically more accurate.

Below are listed the results of error calculations, caused by stochastic nature of the process of secondary photons radiation as applied to investigations of biological object under following conditions:

- channel length of first and second collimators - 5 cm,
- diameter of channels of the first collimator and width of slots of the second collimator - 1 mm, i.e. dimensions of separate volume element (resolution element) are of the order of 1mm $\times$ 1 mm $\times$ 1 mm,
- layer is investigated with a thickness equal to one resolution element, which has dimensions of 5cm × 5cm × 1mm, with distance from central volume element to the outlet of the first collimator and inlet of the second collimator equal to 5 cm (approximately such distances may exist in mammographic investigations),

- radiation energy of X-ray tube - 60 keV.

To achieve mean-root-square error of density determination in each of 2,500 volume elements of layer specified not worse than 3%, radiation dose required amounts to about 0.5 Roentgen. If it is needed to obtain image of such layer in 1 second, then X-ray tube is required with power consumption of the order of 1.5 kW. In calculations, averaged attenuation of primary and excited secondary radiation in biological tissues has been accounted for, coming to about 20%/cm.

Due to the absence of mechanically moved parts, analysis may be performed in shorter time intervals than in the example described, thus allowing to perform it dynamically for objects with a structure changing in time, for example, heart in its different phases and other moving internals of man.

Accuracy of determination of substance density in volume elements of the object investigated is affected also by unequal conditions existing in different volume elements. First of all, primary radiation of X-ray tube 1 suffers unequal attenuation on the way to different volume elements of the object investigated. Secondly, secondary radiation excited in different volume elements of the object investigated, is attenuated to a different extent on the way from these elements to the inlet of corresponding channel of second collimator.

Thus, it is seen from Fig.5, that primary X-ray radiation, coming out of channel 4.5 of the first collimator (cross-hatched horizontal bar 30.5), acts directly on volume element 15.1, penetrating no other volume elements, while radiation coming out of channel 4.7 of the first collimator (horizontal bar 30.7) acts on volume element 15.2 after coming preliminarily through several other volume elements located to the left from said element in the same row. Secondary radiation excited in volume element 15.1 (cross-hatched vertical bar 31.1) directly hits inlet of channel 10.5 of the second collimator, while radiation excited in element 15.2 (vertical bar 31.2) hits inlet of channel 10.6 of the second collimator after penetrating preliminarily several other volume elements located below it in the same column. To increase accuracy of substance density determination in different volume elements of the object investigated, it is expedient to take into account factors specified during generation of numbers proportional to density in unit 19.1 for information processing. For this, it is sufficient to begin calculations from the element 15.1. In this case, for the element located to the right of 15.1 next to it, attenuation of primary radiation may be accounted for using found density value of element 15.1. Similarly, for the next element of the same row, it is possible to take into account attenuation of primary radiation in two preceding elements, whose density has been determined earlier, etc. The same may be made for elements of other rows. At that, it is expedient for the elements of the second row from below, penetrated by primary radiation coming out of channel 4.6 of the first collimator, to take into account attenuation of secondary radiation in its passage to the inlets of channels of the second collimator through elements of the first row, whose density has been determined earlier. Similarly, it is expedient for the elements of the third row from below, penetrated by radiation coming out of channel 4.7, to take into account attenuation of secondary radiation in its passage through elements of second and first rows, etc. for elements in other rows.

For the device according to Fig.3 with slotted channels of the second collimator 9, effects of the factors considered above are illustrated in Fig.6. It is seen from Fig.6 that primary X-ray radiation coming out of channel 4.10 of the first collimator (cross-hatched horizontal bar 32.1) is acting directly on volume element 24.1 of the object investigated without having to pass through other volume elements, while radiation coming out of channel 4.11 of the first collimator (horizontal bar 32.2) affects volume element 24.2 after penetrating previously several other volume elements in the same row. Secondary radiation, excited in volume element 24.1 (hatched sector 33.1) and able to be transmitted to the outlet of slotted channel 23.3 of the second collimator, is coming directly to its inlet, while radiation excited in sector 24.2 (hatched sector 33.2) and able to be transmitted to outlet of slotted channel 23.4 of the second collimator is coming to its inlet after having to penetrate previously several other volume elements situated below in several columns. In Fig.6 such elements exist in two columns: three elements in left column and one element - in right column. These elements are set off with longitudinal hatching.

In order to increase accuracy of substance density determination in different volume elements of the object investigated, it is expedient to take into consideration effects of actual magnitude of attenuation of primary and secondary radiation on the intensity of radiation perceived with detectors. For this, it is expedient to begin generation of numbers proportional to substance density in different volume elements in each of horizontal rows from the volume element located closer to outlet of corresponding channel of the first collimator. For example, in the lower middle row this is element 24.1. For the element most close to it in the way of radiation (in fig.6 - that in front of it, i.e. closer to observer) attenuation of primary radiation may be taken into account using found density value for element 24.1. Similarly, for the next element in this row, attenuation of primary radiation may be taken into account for two preceding elements with density found earlier, etc. The same may be made for elements in other rows of the lower (the closest to the second collimator 9) layer. At that, it is expedient to take into account attenuation of secondary radiation in passing to the inlets of channels of the second collimator through elements of the rows in lower layer with density found earlier. Similarly, for elements belonging to rows of third layer from below, it is expedient to take into account attenuation of secondary radiation in passing through elements of the lower and second layers, etc. for elements of other layers.

Accounting for radiation attenuation (both primary and secondary) is simple and may be accomplished using equation:

$$I_1/I_0 = exp\ [-\mu\,(\rho,\ E) \cdot \Delta x],$$

where $I_0$ and $I_1$ denote, correspondingly, intensity of radiation entering the volume element and exiting from it,

$\Delta x$ is a dimension of volume element in the direction of radiation propagation,

$\mu(\rho,\ E)$ denotes linear photon absorption coefficient dependent on their energy $E$ and mean substance density $\rho$ within the volume element.

In a simplified form, accounting for attenuation of primary and secondary radiation may be realized using *a priori* known mean value of attenuation coefficient for substance density of the object investigated at given radiation energy. Thus, for example, for biological tissues at primary radiation energy *E = 60 keV* mean absorption coefficient $\mu \approx 0,21/cm$. Absorption coefficient of secondary radiation is of the same order, because its photon energy is close to photon energy of primary radiation.

More accurate accounting for attenuation of primary and excited secondary radiation in both cases of the device embodiment considered should be made by taking into consideration of sequential radiation passage through volume elements having different density. Therefore, as it was mentioned earlier, required substance density in the next volume element ought to be determined from radiation intensity after determination of density values in volume elements penetrated by primary radiation on the way to element specified and in elements penetrated by secondary radiation on the way from the next element in line. This prescribes corresponding computational sequence in unit 19.1 of information processing of means 19 for information processing and representation.

The requirement to unit 19.1 implied by such logic consists in its being made with possibility of accounting for attenuation of primary radiation from extended X-ray source on the way to the next in turn volume element of the object investigated, calculated subject to accounting for earlier found values of substance density in other volume elements of the object investigated situated closer to outlet of corresponding channel of the first collimator on intersections of continuation of said channel with continuations of other channels of the second collimator. Unit 19.1 should be made also with possibility of accounting for attenuation of secondary radiation excited in the next in line volume element using found earlier values of substance density in other volume elements of the object investigated situated closer to inlet of the same channel of the second collimator on continuations of said channel with other channels of the first collimator.

If the devices of Fig.1 and Fig.3 are equipped with additional collimators 9a, then in unit 19.1 for information processing similar possibilities should be envisaged for processing of output signals from detectors 17a of additional means 16a for detecting secondary radiation excited in the object investigated.

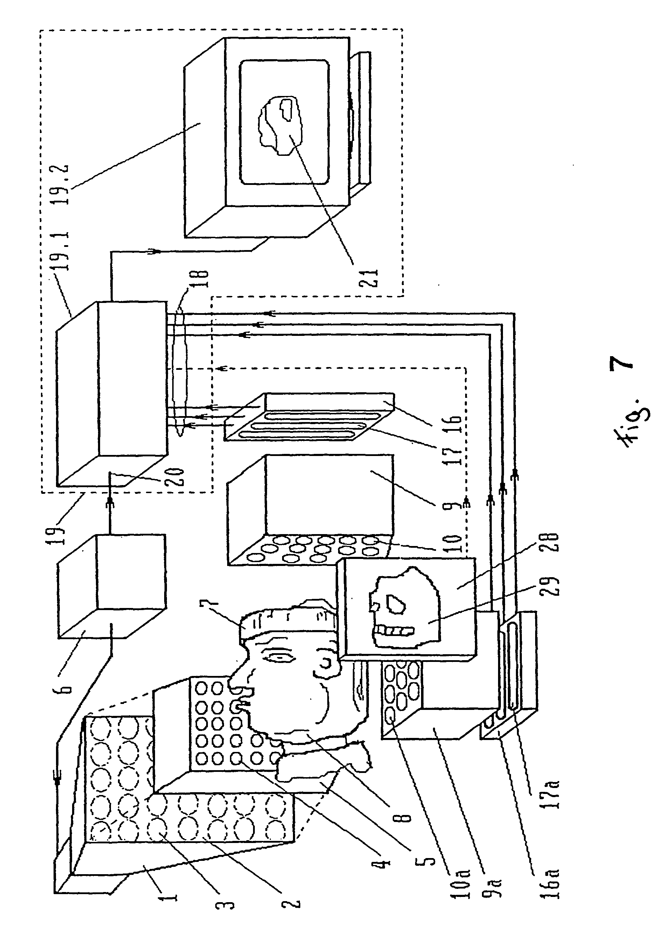

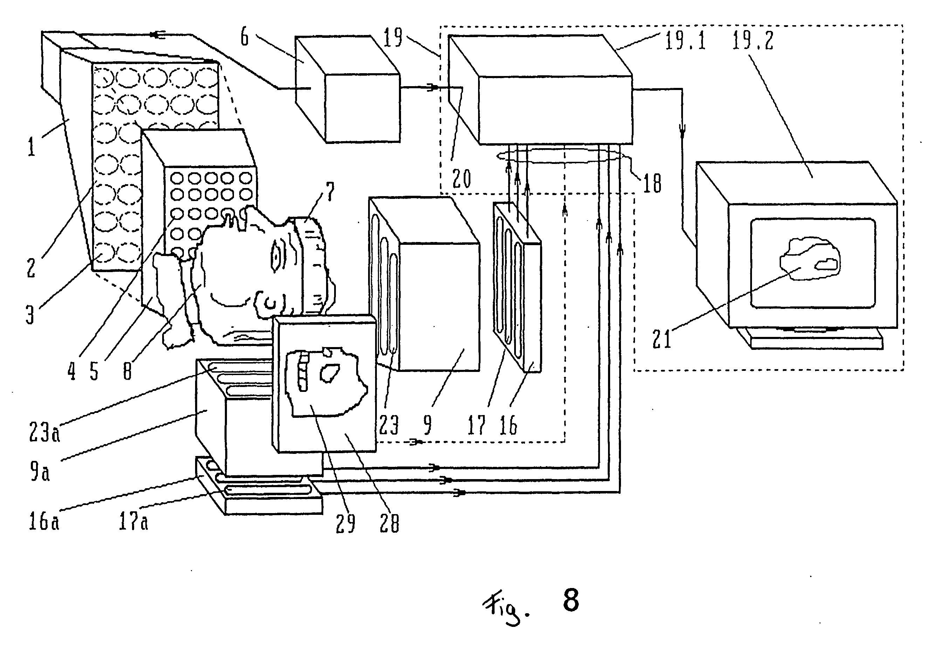

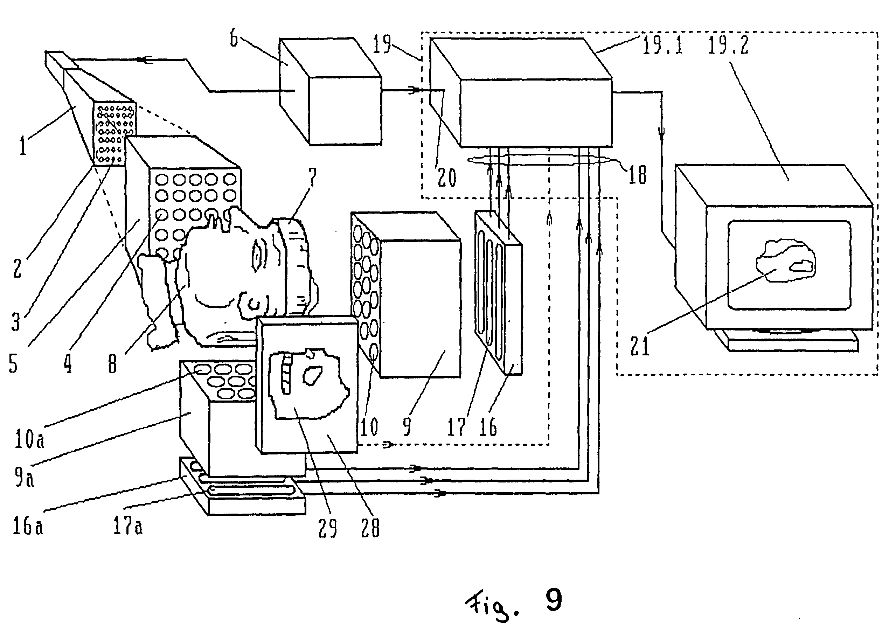

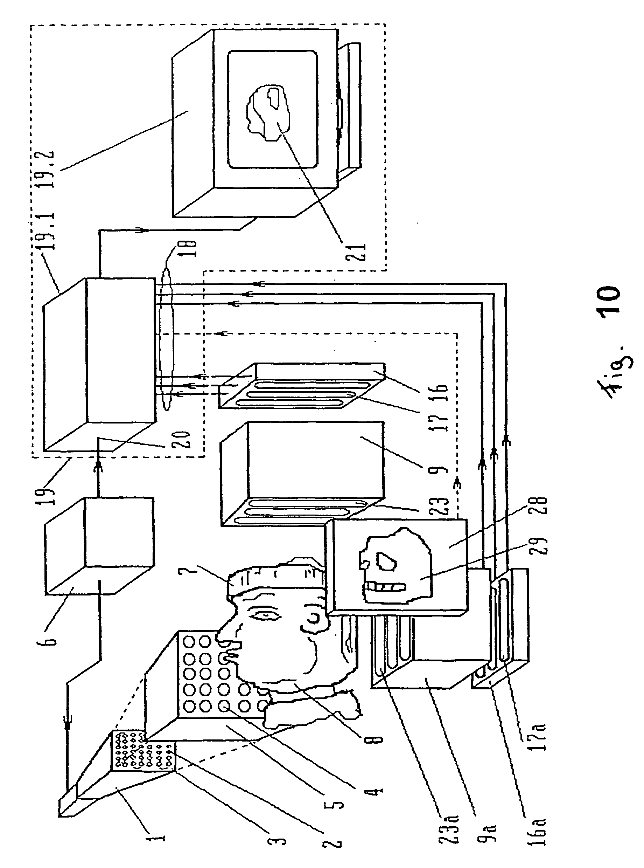

The devices of Fig.1 and Fig.3 may be modified by making first collimators 1 with convergent (Fig. 7, 8) or divergent (Fig. 9, 10) hole-type channels instead of the parallel ones. This allows to perform investigations of objects having dimensions, correspondingly, smaller or bigger than dimensions of anode 2 of tube 1, with one and the same number of resolution elements.

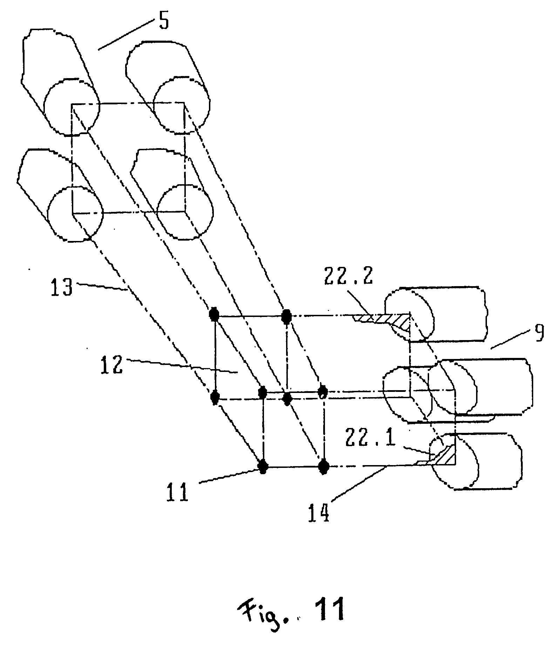

If continuations of centerlines of channels of the first collimator in Fig.7, 8 directed towards the object 8 investigated, and in Fig.9, 10 - towards X-ray tube 1, converge to a point, then cells of imaginary spatial lattice with nodal points contained in the regions of intersection of continuations of channels of first and second collimators have a form of truncated rectangular pyramids. In Fig.11-14 such cells 12 are shown, correspondingly, for the devices of Fig.7-10. If the condition specified above on the convergence of continuations of centerlines of channels of the first collimator in one point is not observed, then cells of imaginary spatial lattice should have a form of other convex hexahedrons (as distinct from rectangular parallelepipeds of Figs.2 and 4, or truncated rectangular pyramids of Figs.11-14), for example, that of obelisk.

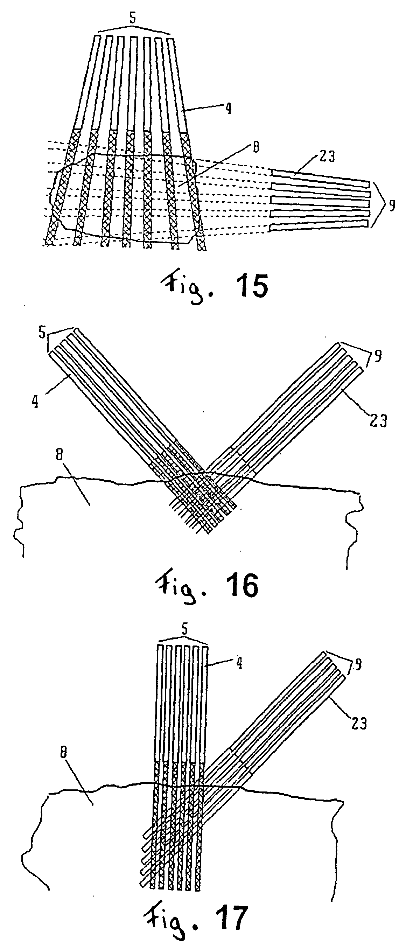

Channels of the second collimator 9 may also be convergent or divergent rather than parallel, and to combine with parallel, divergent or convergent channels of the first collimator 5. See, for example, Fig.15, which shows schematically mutual orientation of channels 4 and 23, correspondingly, of the first 5 and second 9 collimators, diverging towards the object 8 investigated. In all this cases, cells of said imaginary spatial lattice should also have a form of convex hexahedrons.

The possibilities noted for choice of geometry of collimators' channels may be used in specific cases. For example, geometry with divergent channels may be selected in order to ensure full capturing of the object having large dimensions within field of vision of collimators. Geometry with convergent channels may be selected fir investigations of an object having small dimensions without decrease in the number of resolution elements.

Layout of the device is possible with first 5 and second 9 collimators being arranged to one and the same side of the object 8 investigated (Figs.16, 17). As it is seen from Fig.17, channels of the first and second collimators doesn't necessarily have orthogonal mutual orientation.

With the layout shown in Figs.16 and 17, investigations become possible in the cases when for some reasons it is impossible or undesirable to arrange different units of the device at the different sides of the object investigated, for example, in the tasks of industrial defectoscopy of parts of the large-size objects, such as turbines, hulls of ships, walls of active pipelines, and in luggage examination.

The device according to second variant proposed differs from the device embodiment described above in all the particular cases of its embodiment by the absence of link between means 6 for electron beam control and input 20 of means for information processing and representation. Fig.18 shows variant of the device embodiment without said link for the same combination of collimators as in Fig.3. To ensure correct operation of device in the absence of said link, means 6 should be made with additional possibility of such control of tube 1 radiation, which imparts to radiation an attribute carrying information on the current position of electron beam (and, consequently, that of emitting spot 3) on the anode 2 surface. The change specified in the function of means 6 is reflected in Fig.18 by presentation of the link between said means and tube 1 with a double line. The attribute of radiation mentioned may be, for example, frequency of its pulse modulation, pulse duration, combination of these parameters, etc. Unit 19.1 of information processing belonging to means 19 for information processing and representation should be made with additional possibility of extracting information on radiation attribute specified (pulse repetition rate, their duration, etc.) by corresponding demodulation of output signals from detectors 17 of means 16 for detection of secondary radiation excited in the object investigated.

In other aspects, all the above on the device proposed according to the first variant with the drawings illustrating (except for the link specified shown in Figs. 1-3, 7-10) relates in equal measure to the device proposed according to the second variant. Devices according to first and second variants are equivalent in respect of technical result, at the attainment of which are aimed the inventions proposed.

Industrial applicability

Utilization of one or another layout of the device is determined by specific task of analysis of internal structure of the object investigated, setup requirements, possibilities available for manufacturing of one or another type of collimators and other specific circumstances.

Availability of those described and numerous other variants of the proposed device layout offers wide possibilities for development of diagnostic imaging means satisfying specific requirements set for application in medicine, industry, transport and other fields of activity.

Information sources

1. Polytechnical dictionary, Moscow, "Sovetskaya Entsiklopediya" publishing house, 1976 (in Russian).

2. Physics of images visualization in medicine. S. Webb, Ed., Moscow, "Mir" publishing house, 1991 (Russian translation).

3. V.V.Piklov, N.G.Preobrazhensky. Computed tomography and physical experiment. Uspekhi Fizicheskikh Nauk (Progress in Physical Sciences), vol.141, No.3, November 1983 (in Russian).

4. International application PCT/ RU 00/00207, international publication WO 01/59439, 16.08.2001.

5. US patent No.4,259,582 (publ. 31.03.81).

6. US patent No.5,490,197 (publ. 06.02.96).

7. J. Jackson. Classical electrodynamics. Moscow, "Mir" publishing house, 1965 (Russian translation).

8. E.Lapshin. Graphics for IBM PC. Moscow, "Solon" publishing house, 1995 (in Russian).

9. Russian Federation patent No.2,171,979 (publ. 10.08.2001).

10. V.M.Andreevsky, M.V.Gubarev, P.I.Zhidkin, M. A.Kumakhov, A.V.Noshkin, I.Yu.Ponomarev, Kh.Z. Ustok. X-ray waveguide system with a variable cross-section of the sections. The IV-th All Union Conference on Interaction of radiation with Solids (May 15-19, 1990, Elbrus settlement, Kabardino-Balkarian ASSR, USSR). Book of abstracts. Moscow, 1990, pp. 177-178.

11. Chan H.-P., Frank P.H., Doi K., Iida N., Higashida Y. Ultra-High-Strip-Density Radiographic Grids: A New Antiscatter Technique for Mammography. «Radiology», volume 154, Number 3, March 1985, pp. 807-815.

**Claims**

1. Device for imaging of internal structure of the object using X-ray radiation, comprising extended X-ray source, means (7) for positioning of the object (8) investigated, means (16) of detecting secondary radiation excited in the object investigated, means (19) for information processing and representation made with possibility of substance density determination in different parts of volume of the object investigated and displaying of substance density distribution in the object, with output of means (16) of detecting secondary radiation excited in the object investigated connected to the input (18) of latter means, and two multichannel collimators, of which one (5) is situated between the extended X-ray source and the means for positioning of the object investigated, and the other (9) - between said means and means of detecting secondary radiation excited in the object investigated, **characterized in that** the extended X-ray source is a X-ray tube (1) with planar through-target anode (2) made with possibility of emitting spot (3) formation in turn in front of inlets of different channels of the first collimator by corresponding scanning of electron beam of X-ray tube with control means (6) of said beam, having output of data signal on the current position of electron beam connected to second input (20) of means (19) for information processing and representation, said first collimator (5) being an aggregate of parallel, convergent or divergent hole-type channels (4), said second collimator (9) being an aggregate of parallel, convergent or divergent hole-type (10) or slotted (23) channels, both collimators being made and oriented in such a way that continuations of channels of said first collimator intersect with continuations of channels of said second collimator in a zone intended for positioning of the object investigated, thus forming intersection regions comprising nodal points (11, 25) of imaginary spatial lattice with cells (12) in the form of convex hexahedrons, said means (16) of detecting secondary radiation excited in the object investigated being an aggregate of detectors (17) made and mounted each with possibility of reception of output radiation from one only channel of second collimator from the number of those having it at the output simultaneously, and said means (19) for information procession and representation made with possibility of data acquisition on substance density in each of volume elements of the object investigated formed by intersection region of continuations of channels of said first and said second collimators comprising one only of nodal points of said imaginary spatial lattice, from intensity of secondary radiation excited in volume element specified of the object investigated in the course of its irradiation registered by corresponding detector of means for detecting secondary radiation excited in the object investigated.

2. Device according to claim 1, **characterized in that** the channels (10, 23) of second collimator are made parallel to one another, the cells (12) of said imaginary spatial lattice having form of parallelepipeds or rectilinear truncated pyramids.

3. Device according to claims 1 or 2, **characterized**

**in that** the means for information processing and representation is made with possibility of accounting for attenuation of radiation from extended X-ray source on the way to said volume element of the object investigated, computed by accounting for earlier determined values of substance density in other volume elements of the object investigated, situated closer to outlet of corresponding channel of first collimator on intersections of continuation of said channel with continuations of other channels of second collimator.

4. Device according to claim 3, **characterized in that** the means for information processing and representation is made with possibility of accounting for attenuation of said secondary radiation in the course of its passing through the object investigated in the direction of corresponding detector, computed by accounting for earlier determined values of substance density in other volume elements of the object investigated, situated closer to inlet of the same channel of second collimator on intersections of continuation of said channel with continuations of other channels of first collimator.

5. Device according to any of claims 1, 2, and 4, **characterized in that** it comprises at least one additional collimator (9a), made and positioned with adherence to the same conditions as for second collimator, and corresponding additional means (16a) of detecting secondary radiation excited in the object investigated, located near the outlets of channels of additional collimator and connected to the means for information processing and representation made with possibility of the same processing of output signals from detectors of additional means for detecting secondary radiation, as for output signals from detectors of the means for detecting secondary radiation located near outlets of channels of second collimator, and with possibility of generating values of substance density in different volume elements of the object investigated, using as earlier determined values of substance density in other volume elements of the object investigated the values found by co-processing output signals of second and all additional collimators.

6. Device according to claim 5, **characterized in that** the additional collimator (9a) is identical to the second one.

7. Device according to any of claims 1, 2, 4, and 6, **characterized in that** it comprises means (28) for detecting radiation from extended X-ray source transmitted through the object investigated, situated beyond the means (7) for positioning of the object investigated.

8. Device according to claim 7, **characterized in that** the means (28) of detecting secondary radiation from extended X-ray source transmitted through the object investigated is made with possibility of generating visible image of shadow projection (29).

9. Device according to claim 7, **characterized in that** the means (28) of detecting secondary radiation from extended X-ray source transmitted through the object investigated has an electric data output connected to the means for information processing and representation (19), the latter being made with additional possibility of generating and displaying shadow projection image of the object investigated.

10. Device for imaging of internal structure of the object using X-ray radiation, comprising extended X-ray source, means (7) for positioning of the object (8) investigated, means (16) of detecting secondary radiation excited in the object investigated, means (19) for information processing and representation made with possibility of substance density determination in different parts of volume of the object investigated and displaying of substance density distribution within the object, with output of means (16) of detecting secondary radiation excited in the object investigated connected to the input (18) of latter means, and two multichannel collimators, of which one (5) is situated between the extended X-ray source and the means for positioning of the object investigated, and the other (9) - between said means and means of detecting secondary radiation excited in the object investigated, **characterized in that** the extended X-ray source is a X-ray tube (1) with planar through-target anode (2) made with possibility of emitting spot (3) formation in turn in front of inlets of different channels of the first collimator and imparting to X-ray radiation of an attribute carrying information on the current position of the emitting spot by corresponding scanning of electron beam of X-ray tube and modulation of radiation by control means (6) of said beam, said first collimator (5) being an aggregate of parallel, convergent or divergent hole-type channels (4), said second collimator (9) being an aggregate of parallel, convergent or divergent hole-type (10) or slotted (23) channels, both collimators being made and oriented in such a way that continuations of channels of said first collimator intersect with continuations of channels of said second collimator in a zone intended for positioning of the object investigated, thus forming intersection regions comprising nodal points (11, 25) of imaginary spatial lattice with cells (12) in the form of convex hexahedrons, said means (16) of detecting secondary radiation excited in the object investigated being an aggregate of detectors (17) made and mounted each with possibility of reception of output radiation from one only channel of second collimator from the number of those having it at the output simultaneously, and said means (19) for information procession and presentation made with possibility of demodulation of output signals from detectors (17) of means (16) of detecting secondary radiation excited in the object investigated and data acquisition on the current position of the emitting spot, and with possibility of data acquisition on substance density in each of volume elements of the object investigated formed by intersection region of continuations of channels of said first and said second collimators comprising one only of nodal points of said imaginary spatial lattice from intensity of secondary radiation excited in volume element specified of the object investigated in the course of its irradiation, registered by corresponding detector of means for detecting secondary radiation excited in the object investigated.

11. Device according to claim 10, **characterized in that** the control means (6) of electron beam is made with possibility of pulse modulation of radiation of X-ray tube (1) with changing at that of repetition frequency or pulse duration in accordance with the current position of electron beam.

12. Device according to claim 11, **characterized in that** the channels (10, 23) of second collimator are made parallel to one another, the cells (12) of said imaginary spatial lattice having form of parallelepipeds or rectilinear truncated pyramids.

13. Device according to claims 11 or 12, **characterized in that** the means for information processing and representation is made with possibility of taking into account attenuation of radiation from extended X-ray source on the way to said volume element of the object investigated, computed by accounting for earlier determined values of substance density in other volume elements of the object investigated, situated closer to outlet of corresponding channel of first collimator on intersections of continuation of said channel with continuations of other channels of second collimator.

14. Device according to claim 13, **characterized in that** the means for information processing and representation is made with possibility of taking into account attenuation of said secondary radiation in the course of its passing through the object investigated in the direction of corresponding detector, computed by accounting for earlier determined values of substance density in other volume elements of the object investigated, situated closer to inlet of the same channel of second collimator on intersections of continuation of said channel with continuations of other channels of first collimator.

**15.** Device according to any of claims 10, 11, 12, and 14, **characterized in that** it comprises at least one additional collimator (9a), made and positioned with adherence to the same conditions as for second collimator, and corresponding additional means (16a) of detecting secondary radiation excited in the object investigated, located near the outlets of channels of additional collimator and connected to the means for information processing and representation, made with possibility of the same processing of output signals from detectors of additional means for detecting secondary radiation, as for output signals from detectors of the means for detecting secondary radiation located near outlets of channels of second collimator, and with possibility of generating values of substance density in different volume elements of the object investigated, using as earlier determined values of substance density in other volume elements of the object investigated the values found by co-processing output signals of second and all additional collimators.

**16.** Device according to claim 15, **characterized in that** the additional collimator (9a) is identical to the second one.

**17.** Device according to any of claims 10, 11, 12, 14, and 16, **characterized in that** it comprises means (28) for detecting radiation from extended X-ray source transmitted through the object investigated, situated beyond the means (7) for positioning of the object investigated.

**18.** Device according to claim 17, **characterized in that** the means (28) of detecting secondary radiation from extended X-ray source transmitted through the object investigated is made with possibility of generating visible image of shadow projection (29).

**19.** Device according to claim 17, **characterized in that** the means (28) of detecting secondary radiation from extended X-ray source transmitted through the object investigated has an electric data output connected to the means for information processing and representation (19), the latter being made with additional possibility of generating and displaying shadow projection image of the object investigated.

Fig. 1

Fig. 2

Fig. 3

Fig. 4

Fig. 5

Fig. 6

Fig. 7

EP 1 482 305 A1

Fig. 8

Fig. 9

EP 1 482 305 A1

Fig. 10

Fig. 11

Fig. 12

Fig. 13

Fig. 14

EP 1 482 305 A1

Fig. 15

Fig. 16

Fig. 17

Fig. 18

**EP 1 482 305 A1**

<table>
<tr><td colspan="2" align="center">**INTERNATIONAL SEARCH REPORT**</td><td>International application No.<br>PCT/RU 02/00048</td></tr>
</table>

A.  CLASSIFICATION OF SUBJECT MATTER        G01N 23/223

According to International Patent Classification (IPC) or to both national classification and IPC

B.  FIELDS SEARCHED

Minimum documentation searched (classification system followed by classification symbols)

G01N 23/083, 23/20, 23/223, H05G 1/70, 1/52

Documentation searched other than minimum documentation to the extent that such documents are included in the fields searched

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| A | WO 01/59439 A1 (KUMAKHOV MURADIN ABUBEKIROVICH) 16 August 2001 (16.08.2001), the abstract, points 1, 8, 11 of claims | 1-19 |
| A | US 4519092 A (RICHARD D. ALBERT) May 21, 1985 | 1-19 |
| A | US 5778039 A (ADVANCES MICRO DEVICES, INC.) Jul. 7, 1998, the claim | 1-19 |
| A | RU 2072515 C1 (AKTSIONERNOE OBSCHESTVO "ELSKORT") 27.01.1997, the claims | 1-19 |

[X]  Further documents are listed in the continuation of Box C.        [ ]  See patent family annex.

| | |
|---|---|
| *  Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>**28 October 2002 (28.10.02)** | Date of mailing of the international search report<br>**31 October 2002 (31.10.02)** |
|---|---|
| Name and mailing address of the ISA/<br>**RU**<br>Facsimile No. | Authorized officer<br><br>Telephone No. |

Form PCT/ISA/210 (second sheet) (July 1992)

33